Europäisches Patentamt

European Patent Office

Office européen des brevets

(19)

(11) Numéro de publication: **0 203 838
B1**

(12) **FASCICULE DE BREVET EUROPEEN**

(45) Date de publication du fascicule du brevet:
31.10.90

(21) Numéro de dépôt: 86400926.1

(22) Date de dépôt: 28.04.86

(51) Int. Cl.⁵: **C09C 1/00**, C08K 3/30,
C09D 7/12, C09D 11/02,
A61K 7/00

(54) **Utilisation de pigments colorés à base de sulfures de terres rares dans des matières plastiques, peintures ou vernis.**

(30) Priorité: 30.04.85 FR 8506546

(43) Date de publication de la demande:
03.12.86 Bulletin 86/49

(45) Mention de la délivrance du brevet:
31.10.90 Bulletin 90/44

(84) Etats contractants désignés:
AT BE CH DE FR GB IT LI LU NL SE

(56) Documents cités:
FR-A- 2 100 551

CHEMICAL ABSTRACTS,
vol. 99, no. 10, 5 septembre 1983, page 275, résumé 75398p, Columbus, Ohio, US; &
JP-A-58 77 547 (INOUE-JAPAX RESEARCH
INC.) 10-05-1983

(73) Titulaire: RHONE-POULENC CHIMIE, 25, quai Paul
Doumer, F-92408 Courbevoie Cédex(FR)

(72) Inventeur: Maestro, Patrick, 27, rue Honoré de Balzac,
F-95470 Fosses(FR)

(74) Mandataire: Dutruc-Rosset, Marie-Claude et al,
RHONE-POULENC INTERSERVICES Service Brevets
Chimie 25, Quai Paul Doumer, F-92408 Courbevoie
Cédex(FR)

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit
être formée par écrit et motivée. Elle n'est réputée formée qu'après paiement de la taxe d'opposition (Art. 99(1) Convention sur le
brevet européen).

**Description**

La présente invention a trait à l'utilisation de pigments colorés à base de terres rares.

Les pigments colorés sont largement utilisés dans de nombreuses industries notamment dans celles des peintures et lasures et des matières plastiques.

Cependant, un des paramètres qui peut limiter considérablement le champ d'application d'un pigment coloré est sa stabilité à haute température. En particulier, leur emploi dans les matières plastiques nécessite qu'il résiste à une température d'au moins 175°C.

Il est connu que le sulfure de cadmium et ses dérivés peuvent être utilisés dans les matières plastiques et également dans les peintures. Toutefois l'usage desdits sulfures se trouve limité du fait de la toxicité du cadmium et leur emploi dans les peintures intérieures domestiques ou dans des revêtements susceptibles d'être au contact des enfants ou de denrées alimentaires se trouve exclu.

Pour pallier les inconvénients précités, il était souhaitable de disposer de pigments colorés compatibles avec les matières plastiques et les peintures, par exemple.

Il a maintenant été trouvé et c'est ce que fait l'objet de la présente invention que des sulfures de terres rares répondant à la formule $Ln_2S_3$ dans laquelle Ln représente au moins un élément pris dans le groupe constitué par les lanthanides et l'yttrium, peuvent être utilisés à titre de pigments colorés dans des matières plastiques thermoplastiques ou thermodurcissables, polymères spéciaux, dans les résines utilisées comme composant des peintures et lasures, dans les vernis à ongle et fards utilisés en cosmétologie.

Certains de ces sulfures de terres rares sont notamment décrits dans le brevet français FR-A 2 100 551 ainsi que des procédés de fabrication de ceux-ci.

Par ailleurs, il était connu, notamment par le brevet japonais JP-A-58-77 547 d'utiliser des sulfures de terres rares en association avec des métaux ou des alliages pour colorer ceux-ci.

La demanderesse a trouvé que les sulfures de terres rares pouvaient être utilisés en tant que pigments colorés en raison de leurs propriétés avantageuses:

- bonne stabilité thermique
- large éventail de couleurs
- insolubilité dans les solvants organiques
- bonne résistance à l'attaque chimique
- bonne résistance à la dégradation U.V.
- bon pouvoir opacifiant
- bonne résistance à la migration de la couleur
- bonne dispersabilité notamment dans les matières plastiques

La gamme de couleurs obtenue conformément à l'invention dépend de la nature du cation métallique et du type de réseau cristallin.

Les pigments colorés à base de terres rares présentent une structure sesquisulfure $Ln_2S_3$ identifiée selon la méthode d'analyse par diffraction à l'aide de rayons X.

On donne ci-après à titre illustratif et non limitatif des exemples de pigments colorés.

Les pigments à base de sulfures de cérium ont une couleur variant du brun au rouge selon les conditions de préparation, en particulier, la température de calcination. Ils sont bruns ou rouge sang selon que l'on ait la phase $Ce_2S_3$ β orthorombique (J.C.P.D.S. 20 269) ou la phase $Ce_2S_3$ γ cubique (J.C.P.D.S. 27 104).

Avec le lanthane, on obtient des composés jaunes correspondant à une structure $La_2S_3$ cubique (J.C.P.D.S. 25 1041).

La coloration verte peut être obtenue avec le néodyme et vert-jaune avec le praséodyme. Ils présentent respectivement la structure $Nd_2S_3$ cubique (J.C.P.D.S. 26 1450) et la structure $Pr_2S_3$ cubique (J.C.P.D.S. 27 481).

On dispose d'un composé jaune-marron avec le dysprosium de structure $Dy_2S_3$ cubique (J.C.P.D.S. 26 594).

Différentes nuances de marron peuvent être obtenues: ocre avec le terbium, structure $Tb_2S_3$ cubique brun avec l'erbium, structure $Er_2S_3$ monoclinique (J.C.P.D.S. 21 324) et beige foncé avec l'yttrium, structure $Y_2S_3$ monoclinique (J.C.P.D.S. 22 996).

On donne d'autres exemples de couleur obtenue: brun-gris avec le samarium, structure $Sm_2S_3$ cubique (J.C.P.D.S. 26 1480), brun-vert avec le gadolinium, structure $Gd_2S_3$ γ cubique (J.C.P.D.S. 26 1424), vert-or avec le thulium, structure $Tm_2S_3$ monoclinique (J.C.P.D.S. 30 1364).

Les sulfures de terres rares sont des produits connus et décrits dans la littérature (voir brevet français Fr-A 2 100 551 par exemple).

Leur mode de préparation n'est en aucun cas critique. Ils peuvent être fabriqués selon une voie sèche ou humide.

On donne, ci-après, à titre illustratif et non pas limitatif, un procédé d'obtention des sulfures de terres rares.

Ledit procédé consiste à faire réagir au moins un oxyde d'une terre rare avec un courant gazeux d'hydrogène sulfuré à une température comprise entre 800°C et 1500°C.

Comme oxydes de terres rares, on utilise le peroxyde de cérium ou les sesquioxydes des autres métaux de terres rares ou leur mélange.

Il est souhaitable que l'oxyde mis en œuvre soit d'une bonne pureté au moins 95% et de préférence supérieure à 99% et qu'il soit sec: sa teneur en eau doit être inférieure à 5% et, de préférence, inférieure à 1%.

La mise en œuvre de cet oxyde peut nécessiter une étape de séchage à une température comprise le plus souvent entre 100 et 500°C qui peut être faite séparément ou qui peut être réalisée antérieurement au traitement thermique à haute température.

L'hydrogène sulfuré utilisé présente également une haute pureté. On peut faire appel au produit du commerce qui présente une pureté supérieure à 99% en volume.

La quantité d'hydrogène sulfuré mise en œuvre peut varier dans de larges limites. Généralement, elle est au moins égale à la quantité molaire stoechiométrique et peut être en large excès atteignant 2000% de la quantité stoechiométrique.

Selon un mode de réalisation pratique, on commence à chauffer l'oxyde de terres rares à une température comprise entre 100°C et 500°C et de préférence entre 100 et 150°C.

Le chauffage de l'oxyde est réalisé sous balayage de gaz inertes; azote ou gaz rare notamment l'argon.

Cette opération qui permet de chasser l'air résiduaire et l'eau, dure entre 30 minutes et 2 heures.

Ensuite, on établit le courant gazeux d'hydrogène sulfuré à un débit qui est calculé, sachant que 22,4 litres de gaz correspondent à 1 mole d'hydrogène sulfuré, de telle sorte que l'on ait le rapport avec le ou les oxydes de terres rares précédemment définis.

On poursuit ensuite la montée en température afin d'obtenir une température comprise entre 800°C et 1500°C que l'on maintient pendant une durée variant entre 1 et 10 heures. D'une manière préférentielle, on travaille à une température comprise entre 900°C et 1200°C; cette température étant tenue constante environ 2 à 3 heures.

On procède ensuite au refroidissement de la masse réactionnelle qui peut être réalisé sous atmosphère de gaz sulfurant ou sous atmosphère de gaz inerte.

Après refroidissement, on peut réaliser le broyage du produit obtenu de telle sorte qu'il présente des dimensions de particules allant de 0,1 à 10 μm et de préférence de 0,5 à 2 μm.

Le broyage peut être accompagné d'une opération de sélection granulométrique qui peut être réalisée simultanément ou successivement.

Ce procédé peut être mis en œuvre dans un appareillage classique.

Dans le cas de la mise en œuvre d'un mélange d'oxydes, on peut effectuer un mélange homogène desdits oxydes par exemple, dans un broyeur à billes ou à boulets.

L'opération de sulfuration est réalisée dans un creuset placé dans un four à chambre étanche de préférence tubulaire couplé d'un dispositif permettant de réguler la température et d'un dispositif permettant d'assurer la régulation de débit d'un ou plusieurs gaz susceptibles d'être utilisés au cours du procédé de fabrication des sulfures de terres rares.

Le creuset doit être constitué d'un matériau résistant aux hautes températures et à l'atmosphère sulfurée. D'une manière préférentielle, on utilise un creuset en carbone vitreux, en alumine ou en platine.

Après calcination, le produit peut être soumis à des opérations de broyage réalisées dans le même type de broyeur que celui utilisé lors du mélange et de sélection granulomètrique effectuée par exemple, sur un tamis vibrant. On peut également faire les opérations de broyage et de sélection granulomètrique dans un seul appareillage tel qu'un microniseur.

Un domaine d'application privilégié sulfures de terres rares est la coloration des matières plastiques.

Ils peuvent ainsi être utilisés dans la coloration des matières plastiques, thermoplastiques ou thermodurcissables.

Comme résines thermoplastiques susceptibles d'être colorées selon l'invention, on peut nommer à titre illustratif le chlorure de polyvinyle, l'alcool polyvinylique, le polystyrène, les copolymères styrène-butatiène, styrène-acrylonitrile, acrylonitrile-butadiène-styrène (A.B.S.), les polymères acryliques notamment le polyméthacrylate de méthyle, les polyoléfines tels que le polyéthylène, le polypropylène, le polybutène, le polyméthylpentène, les dérivés cellulosiques par exemple l'acétate de cellulose, l'acétobutyrate de cellulose, l'éthylcellulose, les polyamides dont le polyamide 6–6.

Pour ce qui est des résines thermodurcissables pour lesquelles conviennent les pigments colorés à base de terres rares, on peut citer, par exemple, les phénoplastes, les aminoplastes notamment les copolymères urée-formol, mélamine-formol, les résines époxy et les polyesters thermodurcissables.

On peut également mettre en œuvre ces pigments dans les polymères spéciaux tels que les polymères fluorés en particulier le polytétrafluoréthylène (P.T.F.E.), les polycarbonates, les élastomères silicones, les polyimides.

Dans cette industrie des matières plastiques, on peut mettre en œuvre ces pigments sous forme de poudre.

On peut également les mettre sous une forme pré-dispersée, par exemple en prémélange avec une partie de la résine, sous forme d'un concentré pâte ou d'un liquide ce qui permet de les introduire à n'importe quel stade de la fabrication de la résine.

Les pigments peuvent être incorporés dans les matières plastiques en une proportion pondérale de 0,01 à 5%.

Les sulfures de terres rares peuvent être utilisés à titre de pigments colorés dans le domaine des peintures et lasures et plus particulièrement dans les résines suivantes: résines alkydes dont la plus courante est dénommée glycérophtalique; les résines modifiées à l'huile longue ou courte; les résines acryliques dérivées des esters de l'acide acrylique (méthylique ou éthylique) et méthacrylique éventuellement copolymérisés avec l'acrylate d'éthyle, d'éthyl-2 hexyle ou de butyle; les résines vinyliques comme par exemple l'acétate de polyvinyle, le chlorure de polyvinyle, le butyral polyvinylique, le formalpolyvinylique, et les copolymères chlorure de vinyle et acétate de vinyle ou chlorure de vinylidène; les résines aminoplastes ou phénoliques le plus souvent modifiées; les résines polyesters; les résines polyuréthanes; les résines époxy; les résines silicones.

Généralement, les pigments sont mis en œuvre à raison de 5 à 30% en poids de la peinture et de 0,1 à 5% en poids du lasure.

Ils peuvent également convenir pour les applications dans l'industrie du caoutchouc notamment dans les revêtements pour sols et dans l'industrie du papier et des encres d'imprimerie, et pour de nombreuses autres utilisations comme par exemple, le finissage des cuirs et dans les revêtements stratifiés pour cuisine.

Ils peuvent encore être utilisés dans les domaine de la cosmétique et notamment dans les vernis à ongles et dans les fards tels que rouges à lèvres, fards secs, fards gras ou fonds de teint.

Ils peuvent donc être mis en œuvre dans les vernis à ongles qui contiennent généralement:

— un agent filmogène à base de nitrocellulose,

— une résine, résine dammer naturelle ou résine synthétique du type formaldéhyde-sulfamide, résine polystyrène, résine polyvinylique etc...,

— un plastifiant par exemple, le phtalate de diéthyle, le phtalate de dibutyle, le phtalate de dioctyle, le tricrésylphosphate, le stéarate de n-butyle, le diacétate de résorcine ou leur mélange,

— un dissolvant tel que l'alcool éthylique, isopropylique, butylique, isobutylique, l'acétate d'éthyle, l'acétate de butyle ou le plus souvent le mélange de ces solvants,

— un diluant, notamment le toluène ou le xylène,

— éventuellement d'autres additifs, parfum ou produit nacrant (flocons de mica enrobés d'oxychlorure de bismuth ou de bioxyde de titane).

On donne ci-après un exemple de compositon-type:

— de 10 à 15% en poids de nitrocellulose
— de 10 à 15% en poids de résine
— de 3 à 5% en poids de plastifiant(s)
— de 3 à 5% en poids de pigment(s)
— q.s.p. 100% en poids de solvant(s)

Généralement les pigments sont broyés dans une masse plastique constituée de nitrocellulose et de plastifiant(s) qui est ensuite mise en solution dans le(s) solvant(s).

Une autre application des sulfures de terres rares est la coloration des rouges à lèvres.

Les pigments sont mis en œuvre le plus souvent à raison d'une concentration pondérale de 5 à 15% exprimée par rapport à la formulation totale qui renferme:

— un excipient formé d'un mélange de divers corps pour assurer la consistance: cire d'abeille, cire de carnauba, ozocérites, paraffine, cires synthétiques ou leur mélange et d'un excipient mou permettant d'ajuster la consistance tel que le beurre de cacao, la vaseline, les huiles blanches hydrogénées par exemple, l'huile de palme, d'arachide ou de ricin,

- divers additifs notamment un parfum ou arome et le myristate d'isopropyle ou le palmitate d'isopropyle qui donne du glissant,

- un solvant intermédiaire pour mettre en suspension le pigment dans la phase lipophile qui peut être l'huile de ricin ou un glycol comme le polyoxyéthylèneglycol 400 ou des esters d'acides gras : monoricinoléate de propylène glycol, myristate d'isopropyle, palmitate d'isopropyle, stéarate de butyle.

On donne, ci-après, à titre illustratif, un exemple de composition de rouge à lévres :
— ozocérite 4% en poids
— cire de carnauba 2% en poids
— cire de candelilla 8% en poids
— mélange de diesters de propylèneglycol d'acides caprylique et caprique 31% en poids
— huile de ricin 24% en poids

– huile de vaseline q.s.p. 100% en poids
– para-oxybenzoate de propyle 0,1% en poids
– tertiobutyl hydroxyanisole 0,1% en poids

– pigment coloré 2,5% en poids
– oxyde de titane 0,5% en poids
– huile de ricin 15,0% en poids
– parfum 0,50% en poids

On fond le mélange des cires à une température supérieure à la température de fusion de la cire la plus élevée. Dans le mélange fondu, on ajoute le pigment en suspension dans le solvant puis le parfum. Enfin le mélange obtenu est coulé dans des moules.

Les fards à yeux et les fards à joues peuvent se présenter sous forme de fards secs ou de fards gras. La teneur en pigments dans de tels fards peut varier dans de larges limites de 5 à 20%.

Les fards secs sont des poudres (talc, carbonate de magnésium, stéarate de zinc) qui sont chargées en pigments et agglomérées soit avec de la méthylcellulose, soit avec des stéarates.

On donne, à titre d'exemple la composition d'un fard à paupières:
– silicate d'aluminium et de magnésium (Veegum® F) 7% en poids
– talc 50% en poids
– oxyde de zinc 4% en poids
– stéarate de zinc 11% en poids
– kaolin 10% en poids
– pigment 18% en poids

Les pigments à base de terres rares peuvent également être employés dans les formulations de fond de teint.

Les fonds de teint se présentent sous forme d'émulsion en général du type huile dans l'eau.

La phase lipophile comprend le plus souvent:

– un composant huileux tel que l'huile de vaseline, des esters d'acides gras et d'alcools éventuellement gras, par exemple, l'oléate d'oléyle, l'oléate de décyle, le stéarate d'octyle, l'adipate de di-n-butyle, le myristrate d'isopropyle, le palmitate d'isopropyle, le stéarate d'isopropyle, les esters d'acides caprique et caprylique d'alcools gras saturés ayant de 12 à 18 atomes de carbone, une huile de silicone ou leur mélange entre eux,

– un agent émulsifiant du type anionique et/ou non-ionique et plus précisément le sels d'acides gras, stéarate de sodium, de potassium ou d'ammonium, palmitate de sodium; les esters de sorbitan et d'acides gras tels que, par exemple, l'acide laurique, l'acide palmitique, l'acide stéarique; les esters polyoxyéthylénés de sorbitan et d'acides gras contenant de 4 à 20 moles d'oxyde d'éthylène par mole d'ester; les alcools gras polyoxyéthylénés contenant de 2 à 23 moles d'oxyde d'éthylène par mole d'alcool, ledit alcool pouvant être notamment l'alcool laurique, l'alcool cétylique, l'alcool stéarylique, l'alcool oléylique; le mono- et distéarate de glycérol, le mono- et dioléate de glycérol; les acides gras poxyloxyéthylénés et en particulier le stéarate polyoxyéthyléné contenant de 18 à 100 moles d'oxyde d'éthylène par mole d'acide,

– un agent permettant d'ajuster la consistance qui peut être un alcool gras ou un acide gras et plus précisément l'alcool cétylique, l'alcool stéarylique, l'acide stéarique.

Quant à la phase hydrophile, elle est constituée d'eau, de préférence distillée et de divers additifs, notamment:

– un agent humectant qui peut être, par exemple, le propylèneglycol, le glycérol, le sorbitol,

– un agent conservateur et plus particulièrement l'o-phénylphénol et le acides suivants, leurs sels (Na, K, $NH_4$) ou leurs esters ayant de 1 à 4 atomes de carbone: l'acide benzoïque, l'acide salicylique, l'acide sorbique, l'acide p-hydroxybenzoïque,

– un agent de stabilité notamment le dérivés cellulosiques dont la carboxyméthylcellulose et la gomme Xanthane.

Un illustration d'une formulation pour fonds de teint est donnée ci-après:

– phase lipophile:
– huile de vaseline 15% en poids
– mono- et distéarate de glycérol 4% en poids
– alcool cétylique 1% en poids
– phase hydrophile:
– eau distillée q.s.p. 100% en poids
– propylèneglycol 3% en poids
– para-oxybenzoate de méthyle 0,05% en poids
– para-oxybenzoate de propyle 0,1% en poids
– pigment coloré 1 à 10% en poids
– oxyde de titane 3% en poids

La préparation des formulations de fonds de teint est conduite en dispersant d'abord le pigment dans la phase lipophile maintenue vers 60–80°C puis la phase hydrophile maintenue à une des températures comprises dans l'intervalle précité est ajoutée sous agitation et lentement dans la phase lipophile.

Dans l'exposé qui précède, il est donné des exemples de formulations destinées à la cosmétique dans lesquelles peuvent convenir les pigments colorés à base de terres rares et il va sans dire que ces exemples de même que les différents composants cités ne présentent aucun caractère limitatif et ne sont donnés qu'à titre illustratif.

Avant de concrétiser par deux exemples, l'aptitude de ces pigments pour les applications dans les matières plastiques et dans le domaine de la cosmétique, on donne des exemples de fabrication de différents sulfures de terres rares qui sont ensuite caractérisés par leur structure cristalline et par leur couleur.

La structure cristalline est déterminée selon la méthode Debye et Scherrer en transmission: rayonnement monochromatique du molybdène.

La couleur est mesurée à l'aide d'un spectro-photocolorimètre utilisant une sphère intégratrice.

La mesure de la couleur est exprimée en système Hunter ou L, a, b. Ce système L, a, b permet de situer les écarts de couleur et fait appel aux grandeurs suivantes L, a, b:

L donne une mesure de la réflectance (nuance claire/sombre) et varie de 100 (blanc) à 0 (noir)

a et b sont les valeurs des tendances colorées:

a positif = rouge

a négatif = vert

b positif = jaune

b négatif = bleu

L représente donc la variation du noir au blanc, a la variation du rouge au vert et b la variation du jaune au bleu.

Les exemples qui suivent, illustrent l'invention sans toutefois la limiter. Dans les exemples, les pourcentages donnés sont exprimés en poids.

## EXEMPLE 1

On réalise la préparation de sulfure de cérium de structure $Ce_2S_3$ $\gamma$ cubique.

Dans une nacelle de carbone vitreux, on place 10 g d'oxyde de cérium à 99,9% de pureté.

On introduit la nacelle dans un four à tube de quartz.

La température est portée à 500°C sous courant d'argon en 30 minutes.

On remplace alors le courant d'argon par un courant d'hydrogène sulfuré du commerce vendu sous la marque MESSER GRIESHEIM et ayant une pureté de 99,3% en volume.

On monte à 1200°C en 30 minutes sous un débit de gaz de 10 litres/heure.

Le débit est maintenu pendant 3 heures à la température de palier.

On laisse refroidir le produit obtenu à l'inertie du four sous courant d'hydrogène sulfuré jusqu'à 500°C puis sous courant d'argon jusqu'à la tempérture ambiante.

On obtient 10,8 g d'un sulfure de cérium.

Le produit obtenu est un sulfure de cérium pur de structure $Ce_2S_3$ $\gamma$ cubique (J.C.P.D.S. 27 104) et de couleur rouge.

Les coordonnées trichromatiques mesurées sur la poudre dans le système Hunter sont les suivantes:

L = 42,9

a = 28,3

b = 13,8

## EXEMPLE 2

On réalise la préparation de sulfure de cérium de structure $Ce_2S_3$ $\beta$ orthorombique.

Dans une nacelle de carbone vitreux, on place 50 g d'oxyde de cérium à 99,9% de pureté.

On introduit la nacelle dans un four à tube de quartz.

La température est portée à 900°C sous courant d'argon.

On soumet le produit à un courant d'hydrogène sulfuré pendant 90 minutes sous un débit de gaz de 22 litres/heure.

On laisse refroidir le produit obtenu à l'inertie du four sous courant d'hydrogène sulfuré jusqu'à 500°C puis sous courant d'argon jusqu'à la tempérture ambiante.

On obtient 54 g d'un sulfure de cérium.

Le produit obtenu et un sulfure de cérium pur de structure $Ce_2S_3$ $\beta$ orthorombique (J.C.P.D.S. 20 269) et de couleur brun-rouge.

Les coordonnées trichromatiques mesurées sur la poudre dans le système Hunter sont les suivantes:

L = 32,3

a = 11,9

b = 2,7

EXAMPLES 3 A 7

On prépare des sulfures d'autres terres rares selon le mode opératoire décrit à l'exemple 1.

On caractérise les poudres obtenues par leurs coordonnées trichromatiques et l'aspect visuel de leur couleur.

Le tableau I suivant résume les résultats obtenus:

### TABLEAU I

| Ex | Nature du sulfure de terre rare | Estimation visuelle de la couleur | Coordonnées trichromatiques | | |
|---|---|---|---|---|---|
| | | | L | a | b |
| 3 | $La_2 S_3$ | jaune | 72.1 | −5.8 | 41.8 |
| 4 | $Pr_2 S_3$ | vert jaune | 60.8 | −5.0 | 37.0 |
| 5 | $Tb_2 S_3$ | ocre | 61.5 | 1.2 | 32.8 |
| 6 | $Dy_2 S_3$ | jaune marron | 64.3 | −0.8 | 31.3 |
| 7 | $Er_2 S_3$ | brun | 62.1 | 6.0 | 13.9 |

Les pigments obtenus peuvent être mis en œuvre pour la coloration des matières plastiques ou des peintures suivant la description des exemples d'application suivants.

### EXEMPLE 8

Dans cet exemple, on réalise la préparatin de bandes d'un copolymère acrylonitrile-butadiène-styrène (A.B.S.) selon un procédé d'extrusion.

1. On effectue la préparation des échantillons à tester selon le protocole opératoire suivant.

100 g de granulés d'un copolymère A.B.S. commercialisé par PCUK sous la marque UGICRAL sont introduits dans un récipient de 250 cm³ en polyéthylène.

On dispose sur ces granulés sans précaution particulière, 0,5 g du pigment préparé selon l'exemple 1.

On ferme le récipient et l'agite manuellement et périodiquement jusqu'à ce que l'ensemble des granulés présente visuellement une coloration régulièrement répartie.

2. On extrude les granulés obtenus, en profilés plats, au travers d'une filière, à l'aide d'une extrudeuse équipée d'une vis cylindrique destinée à fluidifier les granulés d'A.B.S.

L'extrusion est effectuée à l'aide d'un extrusiographe BRABENDER équipé d'une vis de diamètre = 15 mm et de rapport longueur de vis/diamètre = 12.

Le taux de compression est de 1.

Le fourreau est terminé par une filière plate ayant les dimensions suivantes:

− largeur = 2 cm

− épaisseur = 0,5 mm

Les conditions d'extrusion choisies pour les essais sont données ci-après:

− température du fourreau avec une seule zone de chauffe: 190°C

− température de la filière: 210°C

− vitesse de rotation de la vis: 30 tours/minute

On obtient une matière plastique rouge dont les coordonnées trichromatiques sont L = 45,2; a = 20,2 et b = 11,1.

### EXEMPLE 9

On effectue la préparation d'éprouvettes de polyamide 6–6 (acide adipique + hexaméthylène diamine) selon la technique de moulage par injection.

1. On prépare le échantillons à tester de la manière qui suit:

3 kg de granulés de polyamide 6–6 commercialisé par Rhône-Poulenc sous la dénomination Technyl Type A 216 sont introduits dans un sac en polyéthylène.

On dispose sur ces granulés sans précaution particulière, 9 g d'un pigment préparé selon l'exemple 2.

On introduit le sac dans un récipient cubique tournant horizontalement autour de l'un de ses axes.

On met en rotation le cube mélangeur pendant 10 minutes pour disperser le pigment.

2. On confectionne des éprouvettes par moulage sous injection.

Le moulage par injection consiste à fluidifier les granulés de polyamide 6–6 par chauffage et malaxage dans un fourreau de presse cylindrique, à l'aide d'une vis, puis à injecter la matière fondue dans un moule à tempérture convenable en la poussant à l'aide de la même vis servant de piston.

L'injection est effectuée sur presse BULHER-ROVER 100 B.

Le moule utilisé est une éprouvette plate ayant les dimensions suivantes:

– épaisseur = 4 mm
– poids = 134 grammes

Les conditions d'injection choisies pour les essais sont données ci-après:

– température du cylindre:
zone 1: 270°C
zone 2: 275°C
zone 3: 275°C
buse: 282°C
– température du moule: 60°C
– température de la trémie: température ambiante
– pression d'injection: 90 bars (90.10$^5$ Pa)
– pression de maintien: 39 bars (39.10$^5$ Pa)
– contre-pression: nulle
– temps de maintien de la pression: 9 secondes
– durée du cycle total: 1 minute
– vitesse de rotation de la vis: 174 tours/minute

L'éprouvette en matière plastique obtenue est de couleur rouge et de coordonnées trichromatiques: L = 35,8, a = 8,6 et b = 1,8.

## EXEMPLE 10

Cet exemple illustre la mise en œuvre de sulfure de cérium $Ce_2S_3$ γ cubique dans la préparation d'un fond de teint du type émulsion huile dans eau.

On chauffe à 75°C ± 5°C une phase lipophile constituée de:

– 15 g d'huile de vaseline
– 4 g de mono- et distéarate de glycérol (ARLACEL® 165)
– 1 g d'alcool cétylique

On disperse sous agitation 1 g de sulfure de cérium dans la phase lipophile et 3 g d'oxyde de titane.

On ajoute sous agitation une phase hydrophile chauffée à 75°C et contenant:

– 3 g de propylèneglycol
– 0,05 g de para-oxybenzoate de méthyle
– 0,1 g de para-oxybenzoate de propyle
– 20 g d'un sol aqueux à 1% de gomme Xanthane (Rhodopol® 23 SC)
– q.s.p. 100 g d'eau distillée

On maintient, sous agitation, pendant le refroidissement jusqu'à température ambiante.

On obtient une émulsion teintée en rose.

## Revendications

1. Utilisation, à titre de pigments colorés, d'au moins un sulfure de terres rares répondant à la formule $Ln_2S_3$ dans laquelle Ln représente au moins un élément pris dans le groupe constitué par les lanthanides et l'yttrium dans des matières plastiques thermoplastiques ou thermodurcissables, polymères spéciaux, dans les résines utilisées comme composant des peintures et lasures, dans les vernis à ongle et fards utilisés en cosmétologie.

2. Utilisation selon la revendication 1 caractérisée par le fait que le sulfure de terres rares est un sesquisulfure de lanthane, de cérium, de néodyme, de praséodyme, de samarium, de gadolinium, de terbium, de dysprosium, d'erbium, de thulium et d'yttrium.

3. Utilisation selon la revendication 1 ou 2 caractérisée par le fait que les pigments sont mis en œuvre dans des résines thermoplastiques tels que le chlorure de polyvinyle, l'alcool polyvinylique, le polystyrène, les copolymères styrène butadiène, styrène acrylonitrile, acrylonitrile-butadiène-styrène, les polymères acryliques, les polyoléfines, les dérivés cellulosiques, les polyamides.

4. Utilisation selon la revendication 1 ou 2 caractérisée par le fait que les pigments sont mis en œuvre dans des résines thermodurcissables tels que les phénoplastes, les aminoplastes, les résines époxy, les polyesters thermodurcissables.

5. Utilisation selon la revendication 1 ou 2 caractérisée par le fait que les pigments sont mis en œuvre dans des polymères spéciaux tels que les polymères fluorés, les polycarbonates, les élastomères silicones, les polyimides.

6. Utilisation selon l'une des revendications 3 à 5 caractérisée par le fait que les pigments représentent de 0,01 à 5% du poids des matières plastiques.

7. Utilisation selon la revendication 1 ou 2 caractérisée par le fait que les pigments représentent de 5 à 30% du poids de la peinture ou de 0,1 à 5% du poids du lasure.

8. Utilisation selon la revendication 1 ou 2 caractérisée par le fait que les pigments sont mis en œuvre dans les fonds de teint à raison de 1 à 10% en poids.

## Claims

1. Use as coloured pigments of at least one sulphide of rare earths corresponding to the formula $Ln_2S_3$ in which Ln represents at least one element from the group formed by lanthanides and yttrium in thermoplastic or thermosetting plastics materials, special polymers, in resins used as a component of paints and glazes, in nail varnishes and make-up materials used in cosmetology.

2. Use according to claim 1 characterised in that the sulphide of rare earths is a sesquisulphide of lanthanum, cerium, neodymium, praseodymium, samarium, gadolinium, terbium, dysprosium, erbium, thulium and yttrium.

3. Use according to claim 1 or claim 2 characterised in that the pigments are used in thermoplastic resins such as polyvinyl chloride, polyvinyl alcohol, polystyrene, styrene butadiene, styrene-acrylonitrile and acrylonitrile-butadiene-styrene copolymers, acrylic polymers, polyolefins, cellulose derivatives and polyamides.

4. Use according to claim 1 or 2 characterised in that the pigments are used in thermosetting resins such as phenolic resins, aminoplastic resins, epoxy resins and thermosetting polyesters.

5. Use according to claim 1 or claim 2 characterised in that the pigments are used in special polymers such as fluorinated polymers, polycarbonates, silicone elastomers and polyimides.

6. Use according to one of claims 3 to 5 characterised in that the pigments represent from 0.01 to 5% of the weight of the plastics materials.

7. Use according to claim 1 or claim 2 characterised in that the pigments represent from 5 to 30% of the weight of the paint or from 0.1 to 5% of the weight of the glaze.

8. Use according to claim 1 or claim 2 characterised in that the pigments are used in make-up foundations in a proportion of from 1 to 10% by weight.

## Patentansprüche

1. Verwendung von mindestens einem Seltenerdmetallsulfid der Formel $Ln_2S_3$, in der Ln mindestens ein Element bedeutet, das aus der von den Lanthanoiden und Yttrium gebildeten Gruppe ausgewählt ist, als Farbpigmente in Thermo- oder Duroplasten, Spezialpolymeren, in als Bestandteil von Anstrichstoffen und Lasuren verwendeten Harzen, in Nagellacken und in der Kosmetik verwendeten Schminken.

2. Verwendung nach Anspruch 1, dadurch gekennzeichnet, daß das Seltenerdmetallsulfid ein Sesquisulfid des Lanthans, des Cers, des Neodyms, des Praseodyms, des Samariums, des Gadoliniums, des Terbiums, des Dysprosiums, des Erbiums, des Thuliums und des Yttriums ist.

3. Verwendung nach Anspruch 1 oder 2, dadurch gekennzeichnet, daß die Pigmente in Thermoplasten wie Polyvinylchlorid, Polyvinylalkohol, Polystyrol, den Styrol-Butadien-, Styrol-Acrylnitril-, Acrylnitril-Butadien-Styrol-Copolymeren, den Acrylpolymeren, den Polyolefinen, den Cellulosederivaten und den Polyamiden eingesetzt werden.

4. Verwendung nach Anspruch 1 oder 2, dadurch gekennzeichnet, daß die Pigmente in Duroplasten wie den Phenoplasten, den Aminoplasten, den Epoxidharzen und den duroplastischen Polyestern eingesetzt werden.

5. Verwendung nach Anspruch 1 oder 2, dadurch gekennzeichnet, daß die Pigmente in Spezialpolymeren wie den fluorierten Polymeren, den Polycarbonaten, den Silicon-Elastomeren und den Polyimiden eingesetzt werden.

6. Verwendung nach einem der Ansprüche 3 bis 5, dadurch gekennzeichnet, daß die Pigmente 0,01 bis 5 Gew.% der Kunststoffe ausmachen.

7. Verwendung nach Anspruch 1 oder 2, dadurch gekennzeichnet, daß die Pigmente 5 bis 30 Gew.% des Anstrichstoffs oder 0,1 bis 5 Gew.% der Lasur ausmachen.

8. Verwendung nach Anspruch 1 oder 2, dadurch gekennzeichnet, daß die Pigmente in Make-ups in einem Anteil von 1 bis 10 Gew.% eingesetzt werden.